# EUROPÄISCHE PATENTSCHRIFT

(11) **EP 3 685 494 B1**
(45) Veröffentlichungstag und Bekanntmachung des Hinweises auf die Patenterteilung: **27.04.2022**
(21) Anmeldenummer: 18762844.1
(22) Anmeldetag: 04.09.2018
(51) Int. Cl.: H02K 5/136, G01N 33/00

(54) **VERFAHREN UND VORRICHTUNG ZUM SCHUTZ VON BAUELEMENTEN IN EINEM GEHÄUSE**
METHOD AND APPARATUS FOR PROTECTING COMPONENTS IN A HOUSING
PROCÉDÉ ET DISPOSITIF DE PROTECTION DE COMPOSANTS DANS UN BOÎTIER

(30) Priorität: 18.09.2017 AT 507802017
(43) Veröffentlichungstag der Anmeldung: 29.07.2020
(73) Patentinhaber: Taurob GmbH, 1230 Wien (AT)
(72) Erfinder: SILBERBAUER, Lukas-Sebastian, 2380 Perchtoldsdorf (AT)
(74) Vertreter: Gibler & Poth Patentanwälte KG
(86) Internationale Anmeldenummer: PCT/EP2018/073702
(87) Internationale Veröffentlichungsnummer: WO 2019/052851

(56) Entgegenhaltungen:
- DE-A1- 2 808 046

## Beschreibung

Die Erfindung betrifft ein Verfahren gemäß dem Patentanspruch 1.

Es ist bekannt Geräte, welche in explosionsgefährdeten Bereichen eingesetzt werden, mit einem Überdruckgehäuse zu versehen. Das Überdruckgehäuse verfügt über eine Zuleitung eines reaktionsträgen Gases. Durch den Überdruck entweicht das zugeführte Gas durch Undichtheiten des Gehäuses, wodurch Gas aus der Umgebung des Gehäuses nicht in das Gehäuse gelangen kann. Dadurch können in dem Gehäuse Bauelemente betrieben werden, welche mit dem gefährlichen Gas in der Umgebung nicht in Berührung kommen dürfen.

Nachteilig daran ist, dass zum Betreiben derartiger Geräte eine externe Überdruckversorgung notwendig ist, wodurch solche Geräte stationär zu betreiben sind oder in deren Mobilität stark eingeschränkt sind.

Aus der DE 28 08 046 A1 ist ein Verfahren zum Schutz von Bauelementen in einem explosionsgeschütztem Gehäuse bekannt, wobei das Gehäuse gasdicht ist und unter Überdruck steht. Der Überdruck wird von einer Druckgasanlage mittels einer Gasflasche bereitgestellt.

Aufgabe der Erfindung ist es daher, ein Verfahren der eingangs genannten Art anzugeben, mit welchem die genannten Nachteile vermieden werden können, und mit welchem das Gerät unabhängig von einer externen Überdruckversorgung gemacht werden kann.

Erfindungsgemäß wird dies durch die Merkmale des Patentanspruches 1 erreicht. Dadurch ergibt sich der Vorteil, dass das Gerät auch ohne eine externe Überdruckversorgung oder eines endlichen, lokalen Druckluftspeichers betrieben werden kann. Hierbei kann das zur Aufrechterhaltung des Überdruckes benötigte Gas aus der Umgebung des Gehäuses entnommen werden, wobei sichergestellt werden kann, dass das Gas für den Betrieb des Gerätes ungefährlich ist. Dies wird dadurch erreicht, dass lediglich von dem wenigstens einen Gassensor geprüftes, und ein Ungefährlichkeitskriterium erfüllendes Gas in das Gehäuse eingeleitet wird. Hierbei ist bei einer Vielzahl an Anwendungsgebieten üblicherweise das Gas in der Umgebung frei von gefährlichen Bestandteilen, und ein Schutz vor gefährlichen Gasen lediglich aus Sicherheitsgründen notwendig. Weiters hat sich gezeigt, dass bei vielen Anwendungsgebieten gefährliche Gase lediglich temporär oder lokal auftreten, wodurch ein Ausgleich des Druckverlustes unregelmäßig erfolgen kann. Dadurch wird der Betrieb des Gerätes wesentlich vereinfacht und es werden auch
[weiter auf Seite 2 der ursprünglichen Beschreibung] neue Verwendungen des Gerätes möglich, beispielsweise als ATEX zertifizierter Überwachungsroboter auf einer Bohrinsel.

Weiters betrifft die Erfindung ein Gerät gemäß dem Oberbegriff des Patentanspruches 8.

Aufgabe der Erfindung ist es daher weiters ein Gerät der eingangs genannten Art anzugeben, mit welchem die genannten Nachteile vermieden werden können, und welches unabhängig von einer externen Überdruckversorgung betrieben werden kann.

Erfindungsgemäß wird dies durch die Merkmale des Patentanspruches 8 erreicht.

Die Vorteile des Gerätes entsprechen den Vorteilen des Verfahrens.

Die Unteransprüche betreffen weitere vorteilhafte Ausgestaltungen der Erfindung.

Ausdrücklich wird hiermit auf den Wortlaut der Patentansprüche Bezug genommen, wodurch die Ansprüche an dieser Stelle durch Bezugnahme in die Beschreibung eingefügt sind und als wörtlich wiedergegeben gelten.

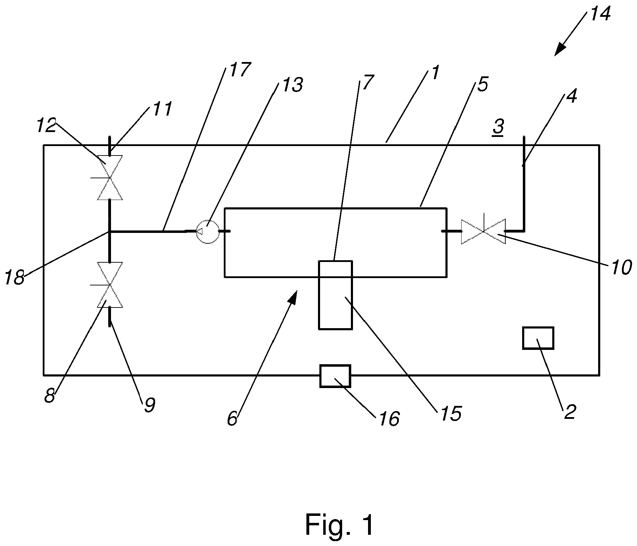

Die Erfindung wird unter Bezugnahme auf die beigeschlossene Zeichnung, in welcher lediglich eine bevorzugte Ausführungsform beispielhaft dargestellt ist, näher beschrieben. Dabei zeigt Fig. 1 eine bevorzugte Ausführungsform des Gerätes als schematische Darstellung.

Die Fig. 1 zeigt eine bevorzugte Ausführungsform eines Gerätes 14 mit einem Gehäuse 1 und in dem Gehäuse 1 angeordneten Bauelementen 2, wobei das Gehäuse 1 ausgebildet ist Gas mit einem gegenüber einer Umgebung 3 erhöhten Druck aufzunehmen. Das Gehäuse 1 ist dazu vorgesehen gefährliche Bestandteile eines Gases in der Umgebung 3 von den Bauelementen 2 in dem Gehäuse 1 fernzuhalten. Zu diesem Zweck wird in dem Gehäuse 1 ein Überdruck gegenüber der Umgebung 3 aufgebaut, sodass sich in Undichtheiten des Gehäuses 1 ein Gasstrom von einem Inneren des Gehäuses 1 in die Umgebung 3 ausbildet. Dieser Vorgang kann als Druckbeaufschlagung des Gehäuses 1 bezeichnet werden. Die gefährlichen Bestandteile des Gases können insbesondere brennbar und/oder korrosiv und/oder giftig sein. Die Bauelemente 2 können elektrische Bauelemente sein, welche insbesondere die Gefahr aufweisen ein brennbares Gas zu entzünden. Das Gehäuse 1 kann insbesondere als Überdruckgehäuse gemäß der Norm DIN EN 60079-2 oder der ATEX Richtlinie: 2014/34/EU ausgebildet sein. Eine Leckrate des Gehäuses 1 kann insbesondere kleiner 10⁻³ mbar l/s sein. Undichtheiten des Gehäuses 1 können insbesondere durch das Gehäuse durchdringende bewegliche Teile des Gerätes 14 sein.

Vorgesehen ist, dass das Gerät 14 eine Messeinrichtung 6 mit einer Messkammer 5 aufweist, dass die Messkammer 5 über eine Gaseinlassleitung 4 mit der Umgebung 3 des Gehäuses 1 strömungstechnisch verbunden ist, dass in der Messkammer 5 wenigstens ein Gassensor 7 zur Detektion vorgebbarer gefährliche Bestandteile eines Gases angeordnet ist, wobei eine Auswerteeinheit 15 der Messeinrichtung 6 ein Messergebnis des wenigstens einen Gassensors 7 mit einem vorgegebenen Ungefährlichkeitskriterium vergleicht. Die Messeinrichtung 6 ist dazu vorgesehen Gas aus der Umgebung 3 des Gehäuses 1 auf Gefährlichkeit zu prüfen. Hierzu weist die Messeinrichtung 6 eine Messkammer 5 mit wenigstens einen Gassensor 7 auf. Die Messkammer 5 kann insbesondere als Druckbehälter ausgebildet sein. Über die Gaseinlassleitung 4 wird Gas aus der Umgebung 3 in die Messkammer 5 geleitet und anschließend von dem wenigstens einen Gassensor 7 vermessen. Strömungstechnisch verbunden bedeutet in diesem Zusammenhang eine Verbindung in Hinblick auf eine ermöglichte Gasströmung von der Umgebung 3 in die Messkammer 5. Die Gaseinlassleitung 4 ist mit einem ersten Ende gegenüber der Umgebung 3 offen und mit einem zweiten Ende mit der Messkammer 5 verbunden.

Um zu Bestimmen, ob ein Gas gefährlich ist oder nicht, wird ein Ungefährlichkeitskriterium in einer Auswerteeinheit 15 abgespeichert. Das Ungefährlichkeitskriterium kann insbesondere das Vorhandensein und/oder die Menge an bestimmten Bestandteilen des Gases aufweisen, welche nicht vorhanden sein und/oder überschritten werden dürfen. Durch einen Vergleich des Messergebnisses des wenigstens einen Gassensors 7 mit dem Ungefährlichkeitskriterium kann zwischen einem gefährlichen Gas und einem ungefährlichen Gas unterschieden werden. Die gefährlichen Bestandteile des Gases können insbesondere solche sein, welche schwer oder nicht aus der Luft zu filtern sind, wie insbesondere leichte Kohlenwasserstoffe wie Methan oder andere Alkane.

Weiters ist vorgesehen, dass zum Ausgleich eines Druckverlustes im Gehäuse 1 von der Messkammer 5 eine, mittels eines Ventils 8 verschließbare Gaszuleitung 9 in das Gehäuse 1 führt, wobei das Ventil 8 der Gaszuleitung 9 mittels der Messeinrichtung 6 so gesteuert ist, dass lediglich Gas aus der Messkammer 5 zum Ausgleich des Druckverlustes im Gehäuse 1 verwendet wird, wenn das Ungefährlichkeitskriterium erfüllt ist. Das Ventil 8 kann hierbei schaltungstechnisch mit der Messeinrichtung 6 verbunden sein. Da das Gehäuse 1 nicht komplett gasdicht ist, sondern eine gewisse Leckrate aufweist, muss ein Ausgleich des Druckverlustes erfolgen, um einen dauerhaften Betrieb des Gerätes 1 zu ermöglichen. Für den Ausgleich des Druckverlustes wird Gas aus der Messkammer 5 durch die Gaszuleitung 9 in das Gehäuse 1 geleitet, wobei ein in das Gehäuse 1 führendes Ende der Gaszuleitung 9 offen ist. Der Ausgleich eines Druckverlustes im Gehäuse 1 erfolgt lediglich durch Gas aus der Messkammer 5, welches von der Messeinrichtung 6 auf Ungefährlichkeit geprüft wurde. Um dies zu ermöglichen wird das Ventil 8 der Gaszuleitung 9 derart gesteuert, dass ein Ausgleich des Druckverlustes nur erfolgt, wenn das Gas aus der Messkammer 5 ungefährlich ist und daher das Ungefährlichkeitskriterium erfüllt. Falls das Gas das Ungefährlichkeitskriterium nicht erfüllt, kann bevorzugt das Gas aus der Messkammer 5 wieder in die Umgebung abgelassen werden, und der Vorgang später wiederholt werden.

Weiters ist ein Verfahren zum Schutz von Bauelementen 2 in dem Gehäuse 1 vorgesehen, wobei in dem Gehäuse 1 Gas mit einem gegenüber der Umgebung 3 erhöhten Druck angeordnet ist, wobei Gas aus der Umgebung 3 durch die Gaseinlassleitung 4 in die Messkammer 5 der Messeinrichtung 6 geleitet wird, wobei das Gas in der Messkammer 5 mittels des wenigstens einen Gassensors 7 auf vorgegebene gefährliche Bestandteile geprüft wird, wobei das Messergebnis des wenigstens einen Gassensors 7 mit dem vorgegebenen Ungefährlichkeitskriterium verglichen wird, wobei zum Ausgleich des Druckverlustes im Gehäuse 1 Gas aus der Messkammer 5 durch die, mittels des Ventils 8 verschließbare Gaszuleitung 9 in das Gehäuse 1 geleitet wird, wobei das Ventil 8 der Gaszuleitung 9 durch die Messeinrichtung 6 so gesteuert wird, dass lediglich Gas aus der Messkammer 5 zum Ausgleich des Druckverlustes im Gehäuse 1 verwendet wird, wenn das Ungefährlichkeitskriterium erfüllt ist.

Dadurch ergibt sich der Vorteil, dass das Gerät 14 auch ohne eine externe Überdruckversorgung oder eines endlichen, lokalen Druckluftspeichers betrieben werden kann. Hierbei kann das zur Aufrechterhaltung des Überdruckes benötigte Gas aus der Umgebung 3 des Gehäuses 1 entnommen werden, wobei sichergestellt werden kann, dass das Gas für den Betrieb des Gerätes 14 ungefährlich ist. Dies wird dadurch erreicht, dass lediglich von dem wenigstens einen Gassensor 7 geprüftes, und ein Ungefährlichkeitskriterium erfüllendes Gas in das Gehäuse 1 eingeleitet wird. Hierbei ist bei einer Vielzahl an Anwendungsgebieten üblicherweise das Gas in der Umgebung frei von gefährlichen Bestandteilen, und ein Schutz vor gefährlichen Gasen lediglich aus Sicherheitsgründen notwendig. Weiters hat sich gezeigt, dass bei vielen Anwendungsgebieten gefährliche Gase lediglich temporär oder lokal auftreten, wodurch ein Ausgleich des Druckverlustes unregelmäßig erfolgen kann. Dadurch wird der Betrieb des Gerätes 14 wesentlich vereinfacht und es werden auch neue Verwendungen des Gerätes möglich, beispielsweise als ATEX zertifizierter Überwachungsroboter auf einer Bohrinsel.

Besonders bevorzugt kann vorgesehen sein, dass das Gerät 14 ein unbemanntes Fahrzeug ist. Das Gerät 14 kann hierbei ferngesteuert und/oder autonom betrieben werden. Zur Steuerung des Gerätes 14 kann eine Steuervorrichtung vorgesehen sein. Das Gerät 14 kann hierbei einen, in Fig. 1 nicht dargestellten, Energiespeicher, insbesondere Akkumulator aufweisen. Weiters kann das Gerät 14 eine Antriebseinheit, insbesondere umfassend einen Motor, aufweisen, mit welchem das Gerät 14 bewegt werden kann. Die Antriebseinheit kann insbesondere ein Teil der zu schützenden Bauelemente 2 sein, da hier die Gefahr heißer Flächen oder elektrischer Funken besonders hoch ist.

Der Energiespeicher kann insbesondere mit einer kontaktlosen Ladevorrichtung, insbesondere ein Induktionsladegerät, verbunden sein. Da das Gerät 14 keine externe Druckluftversorgung oder internen Druckluftspeicher benötigt, kann dadurch komplett auf externe Anschlüsse verzichtet werden. Dadurch ist das Gerät 14 besonders gut geeignet über längere Zeiträume wartungsfrei betrieben zu werden.

Insbesondere kann die Verwendung des Gerätes 14 auf einer Förderanlage für fossile Brennstoffe, bevorzugt einer Ölbohrinsel, vorgesehen sein. Hierbei kann das Gerät 14 über lange Zeiträume ohne Wartung in einer explosionsgefährlichen Zone betrieben werden.

Weiters kann vorgesehen sein, dass das Gerät 14 zum stationären Betrieb vorgesehen ist.

Der Gassensor 7 kann entsprechend dem Einsatzzweck des Gerätes 14 ausgebildet sein.

Bei einem Einsatz in einem explosionsgefährdeten Gebiet kann der Gassensor 7 insbesondere zur Detektion brennbarer Bestandteile im Gas ausgebildet sein. Insbesondere kann der Gassensor 7 ein Infrarotsensor, besonders bevorzugt ein PID sein, welcher brennbare Gasbestandteile schnell und zuverlässig erkennen kann.

Weiters können weitere Gassensoren an einer Außenseite des Gehäuses 1 angeordnet sein.

Die Verwendung des Gerätes 14 oder der Einsatz des Verfahrens in einem explosionsgefährdeten Bereich ist besonders vorteilhaft, da eine Entzündung des Gases in dem Gehäuse 1 nicht nur einen Verlust des Gerätes 14, sondern auch der umliegenden Anlage zur Folge hat.

Bevorzugt kann vorgesehen sein, dass die zu schützenden Bauelemente 2 lediglich dann eingeschaltet sind, wenn ein Druckunterschied zwischen dem Gehäuse 1 und der Umgebung 3 einen kritischen Wert überschreitet. Der kritische Wert kann insbesondere mindestens 0,5 mbar betragen.

Bevorzugt kann vorgesehen sein, dass die Messeinrichtung 6, die Ventile 8,10,12 und die Pumpe 13, explosionsgeschützt ausgebildet ist. Die explosionsgeschützte Ausbildung kann insbesondere durch eine druckfeste Kapselung oder Vergusskapselung der explosionsgefährlichen Teile der Messeinrichtung 6, der Ventile 8,10,12 und der Pumpe 13 erfolgen. Dadurch kann das Verfahren auch durchgeführt werden, wenn noch kein ausreichender Druck im Gehäuse 1 gegenüber der Umgebung 3 aufgebaut wurde. Dadurch kann das Verfahren auch dazu benutzt werden den Druck in dem Gehäuse 1 aufzubauen und/oder das Gehäuse 1 vor und/oder nach einem Einsatz zu spülen.

Eine Verwendung des Gerätes 14 in einer Umgebung 3 mit korrosiven und/oder giftigen Bestandteilen des Gases hat im Vergleich zu einem explosionsgefährdeten Bereich die Wirkung, dass derartige Gase von dem Inneren des Gehäuses 1 ferngehalten werden, wodurch die Lebensdauer des Gerätes 14 verlängert wird und eine höhere Sicherheit bei Wartungsarbeiten gegeben ist.

Sicherheitsrelevante Bauteile des Gerätes 14 können insbesondere redundant ausgeführt sein. Beispielsweise können mehrere Gassensoren 7 in der Messkammer 5 angeordnet sein. Weiters kann vorgesehen sein, dass mehrere in Serie angeordnete Ventile 8 bei der Gaszuleitung 9 vorgesehen sind, um aus Sicherheitsgründen eine Redundanz zu haben.

Die Gaseinlassleitung 4 kann weiters einen, in Fig. 1 nicht dargestellten, Filter aufweisen. Dieser Filter kann insbesondere dazu vorgesehen sein, Öl und/oder Feuchtigkeit zu filtern.

Ein Volumen der Messkammer 5 kann insbesondere bis zu 10 ccm, bevorzugt bis zu 100 ccm, besonders bevorzugt bis zu 1.000 ccm, betragen.

Weiters kann vorgesehen sein, dass das Volumen der Messkammer 5 maximal 10.000 ccm, bevorzugt maximal 1.000 ccm, beträgt.

Das Volumen und ein Druck in der Messkammer 5 kann insbesondere bestimmen, welche Gasmenge bei einem Ausgleich des Druckverlustes in das Gehäuse 1 geleitet wird und damit auch die Druckerhöhung beim Ausgleich des Druckes.

Besonders bevorzugt kann vorgesehen sein, dass das Gas aus der Messkammer 5 zumindest mittelbar mittels wenigstens einer Pumpe 13 in das Gehäuse 1 gepumpt wird. Hierbei kann vorgesehen sein, dass die wenigsten eine Pumpe 13 vorgesehen ist um das Gas zumindest mittelbar aus der Messkammer 5 in das Gehäuse 1 zu pumpen. Das zumindest mittelbar bedeutet in diesem Zusammenhang, dass der Fluss des Gases auch indirekt durch einen von der wenigstens einen Pumpe 13 verursachten Druckunterschied verursacht werden kann.

In der Messkammer 5 kann insbesondere ein Drucksensor angeordnet sein.

Bevorzugt kann vorgesehen sein, dass die Gaseinlassleitung 4 vor einem Messvorgang der Messeinrichtung 6 durch ein zweites Ventil 10 verschlossen wird. Weiters kann vorgesehen sein, dass die Gaseinlassleitung 4 durch das von der Messeinrichtung 6 gesteuertes zweites Ventil 10 verschließbar ist. Das zweite Ventil 10 kann insbesondere auch während des Ausgleiches des Druckverlustes geschlossen bleiben. Dadurch kann sichergestellt werden, dass bei der Messung kein weiteres Gas aus der Umgebung 3 in die Messkammer 5 eintreten kann. Weiters kann dadurch die Bestimmung der Bestandteile aus Sicherheitsgründen mehrmals am Gas erfolgen.

Sofern ein zweites Ventil 10 vorhanden ist kann das Ventil 8 der Gaszuleitung 9 bevorzugt auch als erstes Ventil 8 bezeichnet werden.

Hierbei kann vorgesehen sein, dass das Ventil 8 der Gaszuleitung 9 geschlossen ist, wenn das Gas durch die Gaseinlassleitung 4 mit einem offenen zweiten Ventil 10 in die Messkammer 5 geleitet wird. Dann wird das zweite Ventil 10 geschlossen und anschließend der Messvorgang durchgeführt. Sofern der Messvorgang ergibt, dass das Ungefährlichkeitskriterium erfüllt ist, und insbesondere etwaige weitere Kriterien erfüllt sind, wird das Ventil 8 der Gaszuleitung 9 geöffnet und das Gas aus der Messkammer 5 in das Gehäuse 1 geleitet.

Alternativ kann vorgesehen sein, dass die Messkammer 5 schlauchförmig ist, wobei der wenigstens eine Gassensor 7 in einem ersten Ende der Messkammer 5 angeordnet ist und die Gaszuleitung 9 an einem zweiten Ende der Messkammer 5 ist, wobei eine Strömungsstrecke in der Messkammer 5 zwischen dem Gassensor 7 und der Gaszuleitung 9 unter Berücksichtigung einer Strömungsgeschwindigkeit des Gases in der Messkammer 5 ausreichend ist, dass bei einer Detektion eines gefährlichen Gases bei dem wenigstens einen Gassensor 7 das Ventil 8 vor Erreichen des gefährlichen Gases geschlossen werden kann. Diese nicht dargestellte Ausführungsform hat den Vorteil, dass eine Überprüfung der Zusammensetzung des Gases im Wesentlichen kontinuierlich erfolgen kann.

Weiters kann vorgesehen sein, dass wenn das Gas in der Messkammer 5 nicht in das Gehäuse 1 geleitet wird, das Gas in der Messkammer 5 durch eine, insbesondere durch ein drittes Ventil 12 verschließbare, Gasauslassleitung 11 in die Umgebung 3 geleitet wird. Weiters kann vorgesehen sein, dass die Messkammer 5 über die, insbesondere durch ein drittes Ventil 12 verschließbare, Gasauslassleitung 11 mit der Umgebung 3 des Gehäuses 1 strömungstechnisch verbunden ist. Dadurch ergibt sich der Vorteil, dass die Messkammer 5 zwischen zwei Messvorgängen einfach gespült werden kann, wobei das Gas über die Gaseinlassleitung 4 in die Messkammer 5 eintritt und über die Gasauslassleitung 11 wieder in die Umgebung 3 austritt. Durch das dritte Ventil 12 kann ein ungewollter Eintritt von Gas in die Messkammer 5 oder das Gehäuse 1 unterbunden werden.

Insbesondere kann vorgesehen sein, dass die Pumpe 13 in einer Verbindungsleitung 17 angeordnet ist, welche in einer Verzweigung 18 in die Gaszuleitung 9 und die Gasauslassleitung 11 übergeht. Dadurch ist eine Pumpe 13 ausreichend, wobei allerdings das Volumen der Verbindungsleitung 17 zu spülen ist, sofern in einer vorangegangenen Messung das Ungefährlichkeitskriterium nicht erfüllt wurde.

Alternativ kann vorgesehen sein, dass die Gaszuleitung 9 und die Gasauslassleitung 11 über eigene Pumpen 13 verfügen.

Gemäß einer weiteren nicht dargestellten Ausführungsform kann vorgesehen sein, dass die Gaseinlassleitung 4 auch zum Auslass des Gases aus der Messkammer 5 verwendet wird. Hierbei kann insbesondere vorgesehen sein, dass die Messkammer 5 als Zylinder einer als Kolbenpumpe ausgebildeten Pumpe 13 ausgebildet ist, wodurch das Gas einfach in die Messkammer 5 eingesaugt und ausgeblasen werden kann. Durch die Ventile 8,10 der Gaseinlassleitung 4 und der Gaszuleitung 9 kann dann gesteuert werden, ob das Gas in die Umgebung 3 oder das Gehäuse 1 ausgeblasen wird.

Insbesondere kann vorgesehen sein, dass die Messkammer 5 innerhalb des Gehäuses 1 angeordnet ist. Dadurch kann die Messkammer 5 und die Messeinrichtung 6 vor äußeren Beschädigungen geschützt werden.

Besonders bevorzugt kann vorgesehen sein, dass die Pumpe 13 das Gas aus der Messkammer 5 saugt. Weiters kann vorgesehen sein, dass strömungstechnisch eine Saugseite der Pumpe 13 der Messkammer 5 zugewandt ist. Vorteilhaft daran ist, dass ein Druck in der Messkammer 5 maximal einem Druck der Umgebung 3 entspricht, wodurch die Analyse des Gases bei einem Druck der Umgebung 3 durchgeführt werden kann, auf welchen viele Gassensoren 7 optimiert sind. Weiters kann aufgrund des Druckunterschiedes zum Gas im Gehäuse 1 verhindert werden, dass Gas aus der Messkammer 5 in das Gehäuse 1 eindringt. Sofern die gefährlichen Bestandteile des Gases brennbar sind kann weiters dadurch verhindert werden, dass eine Kompression des Gases eine Explosion auslöst.

Die bevorzugte Ausführungsform in Fig. 1 kann daher besonders bevorzugt wie folgt betrieben werden. Über die Gaseinlassleitung 4 wird ein das Gehäuse 1 umgebendes Gas durch das zweite Ventil 10 aufgrund eines durch die Pumpe 13 erzeugten Unterdrucks in die Messkammer 5 gesaugt. Das erste Ventil 8 und das dritte Ventil 12 sind dabei geschlossen. Sobald der Druck in der Messkammer 5 mit dem Umgebungsdruck übereinstimmt wird das zweite Ventil 10 geschlossen, und das Gas mittels des Gassensors 7 untersucht. Sofern das untersuchte Gas das Ungefährlichkeitskriterium erfüllt, und ein Bedarf an einem Ausgleich des Druckes besteht, wird das Ventil 8 der Gaszuleitung 9 geöffnet und das Gas durch die Pumpe 13 in das Innere des Gehäuses 1 gepumpt, wodurch ein Unterdruck in der Messkammer 5 entsteht. Wenn das untersuchte Gas das Ungefährlichkeitskriterium nicht erfüllt, oder ein weiteres Kriterium nicht erfüllt ist, bleibt das Ventil 8 verschlossen und es wird stattdessen das dritte Ventil 13 geöffnet, wodurch das Gas über die Gasauslassleitung 11 nach außen gepumpt wird. Hierbei kann zusätzlich das zweite Ventil 10 zumindest zeitweise geöffnet werden, um die Messkammer 5 zu spülen.

Insbesondere kann vorgesehen sein, dass die Messeinrichtung 6 als ein weiteres Kriterium bestimmt, ob ein Ausgleich des Druckverlustes notwendig ist.

Bevorzugt kann vorgesehen sein, dass das Gerät 14 im Wesentlichen ständig mittels des Gassensors 7 das Gas in der Umgebung 3 analysiert, und lediglich dann den Ausgleich des Druckverlustes im Gehäuses durchführt, wenn dieser erforderlich ist. Hierbei kann es insbesondere eine Aufgabe des Gerätes 14 sein, das Gas in der Umgebung 3 zu analysieren.

Insbesondere kann vorgesehen sein, dass die Messeinrichtung 6 wenigstens einen, einen Druckunterschied zwischen dem Gas in dem Gehäuse 1 und der Umgebung 3 messenden Drucksensor 16 aufweist. Weiters kann vorgesehen sein, dass ein Druckunterschied zwischen Gehäuse 1 und Umgebung 3 gemessen wird, und dass der Ausgleich des Druckverlustes nur dann durchgeführt wird, wenn der gemessene Druckunterschied einen ersten Wert unterschreitet. Dadurch kann die Messeinrichtung 6 erkennen, wann ein Ausgleich des Druckverlustes notwendig ist.

Insbesondere kann vorgesehen sein, dass ein maximaler Druckunterschied zwischen Gehäuse 1 und Umgebung 3 vorgegeben ist. Der maximale Druckunterschied kann insbesondere ein Überdruck im Gehäuse 1 von maximal 20 mbar, bevorzugt maximal 10 mbar, besonders bevorzugt maximal 5 mbar, sein. Es kann vorgesehen sein, dass der erste Wert kleiner als der maximale Druckunterschied ist.

Durch das Volumen und den Druck in der Messkammer 5 ist im Wesentlichen die Gasmenge vorgegeben, welche bei einem Ausgleich des Druckverlustes in das Gehäuse geleitet wird. Durch das Verhältnis des Volumens in der Messkammer 5 sowie dem, im Innenraum des Gehäuses 1 dem Gas zur Verfügung stehenden Volumens ergibt sich eine im Wesentlichen konstante Druckerhöhung.

Insbesondere kann vorgesehen sein, dass durch ein Leiten eines Großteils der in der Messkammer 5 vorhandenen Gasmenge in das Gehäuse 1 der Druckunterschied von dem ersten Wert auf im Wesentlichen den maximalen Druckunterschied angehoben werden kann.

Insbesondere kann vorgesehen sein, dass der erste Wert um wenigstens 10%, insbesondere um wenigstens 20%, besonders bevorzugt um wenigstens 30%, kleiner ist als der maximale Druckunterschied.

Weiters kann vorgesehen sein, dass der erste Wert derart gewählt ist, dass bei einer vorgesehenen Leckrate des Gehäuses 1 ein Ausgleich des Druckunterschiedes erst nach mindestens 1 min, insbesondere mindestens 10 min, besonders bevorzugt mindestens 60 min, erforderlich ist. Dies kann durch eine entsprechende Leckrate des Gehäuses 1 und Volumen der Messkammer 5 eingestellt werden.

Besonders bevorzugt kann vorgesehen sein, dass wenn der gemessene Druckunterschied einen zweiten Wert unterschreitet, und das Gas in der Messkammer 5 das Ungefährlichkeitskriterium nicht erfüllt, das Gehäuse 1 mittels einer Antriebseinrichtung zu einer vorgegebenen Position bewegt wird. Hierbei ist das Gerät 14 als autonomes Fahrzeug ausgebildet, welches über die Antriebseinrichtung bewegt wird. Der zweite Wert kann insbesondere kleiner als der erste Wert sein. Dadurch kann das Gerät 14, wenn der erste Wert unterschritten wird seine Aufgabe zunächst fortsetzen, selbst wenn aufgrund gefährlicher Bestandteile des Gases in der Umgebung 3 ein Ausgleich des Druckverlustes nicht möglich ist. Erst bei Erreichen des zweiten Wertes wird versucht das Gerät 14, und damit auch das Gehäuse 1, zu einer Umgebung 3 zu fahren, bei welcher ein ungefährliches Gas wahrscheinlich ist. Die vorgegebene Position kann insbesondere eine vorgespeicherte Position sein, an welcher ein ungefährliches Gas vermutet werden kann, oder eine letzte Position, wo noch ein ungefährliches Gas detektiert wurde.

Der zweite Wert kann insbesondere ein vorgegebener Schwellenwert sein.

Insbesondere kann vorgesehen sein, dass der zweite Wert um wenigstens 50%, kleiner ist als der maximale Druckunterschied.

Alternativ kann vorgesehen sein, dass der zweite Wert anhand einer errechneten Fahrtzeit von einer aktuellen Position zu der vorgegebenen Position bestimmt wird. Dadurch kann erreicht werden, dass bei einem Erreichen des zweiten Wertes das Gerät 14 die vorgegebene Position erreichen kann.

Bevorzugt kann vorgesehen sein, dass auf der Fahrt zu der vorgegebenen Position die Messeinrichtung 6 das Gas der Umgebung 3 misst. Sollte das Gas wieder das Ungefährlichkeitskriterium erfüllen kann dann der Ausgleich des Druckverlustes durchgeführt werden, und die Fahrt zu der vorgegebenen Position abgebrochen werden.

## Patentansprüche

1. Verfahren zum Schutz von Bauelementen (2) in einem Gehäuse (1), wobei in dem Gehäuse (1) Gas mit einem gegenüber einer Umgebung (3) erhöhten Druck angeordnet ist, **dadurch gekennzeichnet, dass** Gas aus der Umgebung (3) durch eine Gaseinlassleitung (4) in eine Messkammer (5) einer Messeinrichtung (6) geleitet wird, dass das Gas in der Messkammer (5) mittels wenigstens einen Gassensors (7) auf vorgegebene gefährliche Bestandteile geprüft wird, dass ein Messergebnis des wenigstens einen Gassensors (7) mit einem vorgegebenen Ungefährlichkeitskriterium verglichen wird, dass zum Ausgleich eines Druckverlustes im Gehäuse (1) Gas aus der Messkammer (5) durch eine, mittels eines Ventils (8) verschließbare Gaszuleitung (9) in das Gehäuse (1) geleitet wird, dass das Ventil (8) der Gaszuleitung (9) durch die Messeinrichtung (6) so gesteuert wird, dass lediglich Gas aus der Messkammer (5) zum Ausgleich des Druckverlustes im Gehäuse (1) verwendet wird, wenn das Ungefährlichkeitskriterium erfüllt ist.

2. Verfahren nach Anspruch 1, **dadurch gekennzeichnet, dass** die Gaseinlassleitung (4) vor einem Messvorgang der Messeinrichtung (6) durch ein zweites Ventil (10) verschlossen wird.

3. Verfahren nach Anspruch 1 oder 2, **dadurch gekennzeichnet, dass** wenn das Gas in der Messkammer (5) nicht in das Gehäuse (1) geleitet wird, das Gas in der Messkammer (5) durch eine, insbesondere durch ein drittes Ventil (12) verschließbare, Gasauslassleitung (11) in die Umgebung (3) geleitet wird.

4. Verfahren nach einem der Ansprüche 1 bis 3, **dadurch gekennzeichnet, dass** das Gas aus der Messkammer (5) zumindest mittelbar mittels wenigstens einer Pumpe (13) in das Gehäuse (1) gepumpt wird.

5. Verfahren nach Anspruch 4, **dadurch gekennzeichnet, dass** die Pumpe (13) das Gas aus der Messkammer (5) saugt.

6. Verfahren nach einem der Ansprüche 1 bis 5, **dadurch gekennzeichnet, dass** ein Druckunterschied zwischen Gehäuse (1) und Umgebung (3) gemessen wird, und dass der Ausgleich des Druckverlustes nur dann durchgeführt wird, wenn der gemessene Druckunterschied einen ersten Wert unterschreitet.

7. Verfahren nach Anspruch 6, **dadurch gekennzeichnet, dass** wenn der gemessene Druckunterschied einen zweiten Wert unterschreitet, und das Gas in der Messkammer (5) das Ungefährlichkeitskriterium nicht erfüllt, das Gehäuse (1) mittels einer Antriebseinrichtung zu einer vorgegebenen Position bewegt wird.

8. Gerät (14) mit einem Gehäuse (1) und in dem Gehäuse (1) angeordneten Bauelementen (2), wobei das Gehäuse (1) ausgebildet ist Gas mit einem gegenüber einer Umgebung (3) erhöhten Druck aufzunehmen, **dadurch gekennzeichnet, dass** das Gerät (14) eine Messeinrichtung (6) mit einer Messkammer (5) aufweist, dass die Messkammer (5) über eine Gaseinlassleitung (4) mit der Umgebung (3) des Gehäuses (1) strömungstechnisch verbunden ist, dass in der Messkammer (5) wenigstens ein Gassensor (7) zur Detektion vorgebbarer gefährliche Bestandteile eines Gases angeordnet ist, wobei eine Auswerteeinheit (15) der Messeinrichtung (6) ein Messergebnis des wenigstens einen Gassensors (7) mit einem vorgegebenen Ungefährlichkeitskriterium vergleicht, dass zum Ausgleich eines Druckverlustes im Gehäuse (1) von der Messkammer (5) eine, mittels eines Ventils (8) verschließbare Gaszuleitung (9) in das Gehäuse (1) führt, wobei das Ventil (8) der Gaszuleitung (9) mittels der Messeinrichtung (6) so gesteuert ist, dass lediglich Gas aus der Messkammer (5) zum Ausgleich des Druckverlustes im Gehäuse (1) verwendet wird, wenn das Ungefährlichkeitskriterium erfüllt ist.

9. Gerät (14) nach Anspruch 8, **dadurch gekennzeichnet, dass** die Gaseinlassleitung (4) durch ein von der Messeinrichtung (6) gesteuertes zweites Ventil (10) verschließbar ist.

10. Gerät (14) nach Anspruch 8 oder 9, **dadurch gekennzeichnet, dass** die Messkammer (5) über eine, insbesondere durch ein drittes Ventil (12) verschließbare, Gasauslassleitung (11) mit der Umgebung (3) des Gehäuses (1) strömungstechnisch verbunden ist.

11. Gerät (14) nach einem der Ansprüche 8 bis 10, **dadurch gekennzeichnet, dass** wenigsten eine Pumpe (13) vorgesehen ist um das Gas zumindest mittelbar aus der Messkammer (5) in das Gehäuse (1) zu pumpen.

12. Gerät (14) nach Anspruch 11, **dadurch gekennzeichnet, dass** strömungstechnisch eine Saugseite der Pumpe (13) der Messkammer (5) zugewandt ist.

13. Gerät (14) nach einem der Ansprüche 8 bis 12, **dadurch gekennzeichnet, dass** die Messkammer (5) innerhalb des Gehäuses (1) angeordnet ist.

14. Gerät (14) nach einem der Ansprüche 8 bis 13, **dadurch gekennzeichnet, dass** die Messeinrichtung (6) wenigstens einen, einen Druckunterschied zwischen dem Gas in dem Gehäuse (1) und der Umgebung (3) messenden Drucksensor (16) aufweist.

15. Gerät (14) nach einem der Ansprüche 8 bis 14, **dadurch gekennzeichnet, dass** das Gerät (14) ein unbemanntes Fahrzeug ist.

## Claims

1. Method for protecting components (2) in a housing (1), wherein gas is arranged in the housing (1) at a pressure which is elevated with respect to an environment (3), **characterized in that** gas is directed from the environment (3) through a gas inlet line (4) into a measuring chamber (5) of a measuring device (6), **in that** the gas in the measuring chamber (5) is tested for predetermined hazardous constituents by means of at least one gas sensor (7), **in that** a measurement result of the at least one gas sensor (7) is compared with a predetermined non-hazardousness criterion, **in that**, in order to compensate for a pressure loss in the housing (1), gas is directed from the measurement chamber (5) through a gas feed line (9), which can be closed by means of a valve (8), into the housing (1), **in that** the valve (8) of the gas feed line (9) is controlled by the measuring device (6) in such a way that only gas from the measuring chamber (5) is used to compensate for the pressure loss in the housing (1) when the non-hazardousness criterion is fulfilled.

2. Method according to claim 1, **characterized in that** the gas inlet line (4) is closed by a second valve (10) before a measuring operation of the measuring device (6).

3. Method according to claim 1 or 2, **characterized in that** if the gas in the measuring chamber (5) is not conducted into the housing (1), the gas in the measuring chamber (5) is conducted into the environment (3) through a gas outlet line (11) which can be closed in particular by a third valve (12).

4. Method according to one of claims 1 to 3, **characterized in that** the gas is pumped out of the measuring chamber (5) at least indirectly into the housing (1) by means of at least one pump (13).

5. Method according to claim 4, **characterized in that** the pump (13) sucks the gas out of the measuring chamber (5).

6. Method according to one of claims 1 to 5, **characterized in that** a pressure difference between the housing (1) and the environment (3) is measured, and **in that** the compensation of the pressure loss is carried out only if the measured pressure difference falls below a first value.

7. Method according to claim 6, **characterized in that** if the measured pressure difference falls below a second value, and the gas in the measuring chamber (5) does not meet the non-hazardousness criterion, the housing (1) is moved to a predetermined position by means of a drive device.

8. Device (14) having a housing (1) and components (2) arranged in the housing (1), wherein the housing (1) is designed to receive gas at a pressure which is higher than a pressure in the environment (3), **characterized in that** the device (14) has a measuring device (6) having a measuring chamber (5), **in that** the measuring chamber (5) is flow-connected to the environment (3) of the housing (1) via a gas inlet line (4), **in that** at least one gas sensor (7) is arranged in the measuring chamber (5) for detecting predefinable hazardous constituents of a gas, wherein an evaluation unit (15) of the measuring device (6) compares a measurement result of the at least one gas sensor (7) with a predetermined non-hazardousness criterion, **in that**, in order to compensate for a pressure loss in the housing (1) of the measuring chamber (5), a gas feed line (9), which can be closed by means of a valve (8), leads from the measuring chamber (5) into the housing (1), wherein the valve (8) of the gas feed line (9) is controlled by means of the measuring device (6) in such a way that only gas from the measuring chamber (5) is used to compensate for the pressure loss in the housing (1) when the non-hazardousness criterion is fulfilled.

9. Device (14) according to claim 8, **characterized in that** the gas inlet line (4) can be closed by a second valve (10) controlled by the measuring device (6).

10. Device (14) according to claim 8 or 9, **characterized in that** the measuring chamber (5) is flow-connected to the environment (3) of the housing (1) via a gas outlet line (11) which can be closed, in particular by a third valve (12).

11. Device (14) according to one of the claims 8 to 10, **characterized in that** at least one pump (13) is provided to pump the gas at least indirectly from the measuring chamber (5) into the housing (1).

12. Device (14) according to claim 11, **characterized in that,** in terms of flow, a suction side of the pump (13) faces the measuring chamber (5).

13. Device (14) according to one of claims 8 to 12, **characterized in that** the measuring chamber (5) is arranged inside the housing (1).

14. Device (14) according to one of the claims 8 to 13, **characterized in that** the measuring device (6) has at least one pressure sensor (16) measuring a pressure difference between the gas in the housing (1) and the environment (3).

15. Device (14) according to one of claims 8 to 14, **characterized in that** the device (14) is an unmanned vehicle.

## Revendications

1. Procédé pour la protection de composants (2) dans un boîtier (1), dans lequel le boîtier (1) contient du gaz en surpression par rapport à l'environnement (3), **caractérisé en ce que** du gaz provenant de l'environnement (3) est amené par une conduite d'entrée de gaz (4) dans une chambre de mesure (5) d'une installation de mesure (6), **en ce que** le gaz dans la chambre de mesure (5) est vérifié au moyen d'au moins un détecteur de gaz (7) à la recherche de composants dangereux, **en ce qu'**un résultat de mesure de l'au moins un détecteur de gaz (7) est comparé à un critère d'innocuité prédéterminé, **en ce qu'**afin de compenser une perte de pression dans le boîtier (1), du gaz provenant de la chambre de mesure (5) est amené dans le boîtier (1) par une conduite d'arrivée de gaz (9) qui peut être fermée par une vanne (8), **en ce que** la vanne (8) de la conduite d'arrivée de gaz (9) est commandée par l'installation de mesure (6) de telle manière que le gaz provenant de la chambre de mesure (5) ne soit utilisé pour compenser la perte de pression dans le boîtier (1) que si le critère d'innocuité est satisfait.

2. Procédé selon la revendication 1, **caractérisé en ce que** la conduite d'entrée de gaz (4) est fermée par une deuxième vanne (10) avant une opération de mesure de l'installation de mesure (6).

3. Procédé selon la revendication 1 ou 2, **caractérisé en ce que**, si le gaz dans la chambre de mesure (5) n'est pas amené dans le boîtier (1), le gaz dans la chambre de mesure (5) est envoyé dans l'environnement (3) via une troisième conduite de sortie de gaz (11), pouvant notamment être fermée par une troisième vanne (12).

4. Procédé selon l'une des revendications 1 à 3, **caractérisé en ce que** du gaz provenant de la chambre de mesure (5) est pompé au moins indirectement au moyen d'au moins une pompe (13) dans le boîtier (1).

5. Procédé selon la revendication 4, **caractérisé en ce que** la pompe (13) aspire le gaz dans la chambre de mesure (5).

6. Procédé selon l'une des revendications 1 à 5, **caractérisé en ce qu'**une différence de pression entre le boîtier (1) et l'environnement (3) est mesurée et **en ce que** la perte de pression n'est compensée que lorsque la différence de pression mesurée passe en dessous d'une première valeur.

7. Procédé selon la revendication 6, **caractérisé en ce que**, si la différence de pression mesurée passe en dessous d'une deuxième valeur et si le gaz dans la chambre de mesure (5) ne satisfait pas le critère d'innocuité, le boîtier (1) est déplacé vers une position prédéterminée au moyen d'une installation d'entraînement.

8. Appareil (14) avec un boîtier (1) et des composants (2) disposés dans le boîtier (1), lequel boîtier (1) est conçu pour recevoir du gaz en surpression par rapport à un environnement (3), **caractérisé en ce que** l'appareil (14) comprend une installation de mesure (6) avec une chambre de mesure (5), **en ce que** la chambre de mesure (5) communique en vue de la circulation avec l'environnement (3) du boîtier (1) par une conduite d'entrée de gaz (4), **en ce que** la chambre de mesure (5) contient au moins un détecteur de gaz (7) pour détecter des composants dangereux d'un gaz pouvant être prédéterminés, une unité d'analyse (15) de l'installation de mesure (6) comparant un résultat de mesure de l'au moins un détecteur de gaz (7) avec un critère d'innocuité prédéterminé, **en ce que**, pour compenser une perte de pression dans le boîtier (1), une conduite d'arrivée de gaz (9) pouvant être fermée au moyen d'une vanne (8) est prévue de la chambre de mesure (5) au boîtier (1), la vanne (8) de la conduite d'arrivée de gaz (9) étant commandée au moyen de l'installation de mesure (6) de telle manière que le gaz provenant de la chambre de mesure (5) ne soit utilisé pour compenser la perte de pression dans le boîtier (1) que si le critère d'innocuité est satisfait.

9. Appareil (14) selon la revendication 8, **caractérisé en ce que** la conduite d'entrée de gaz (4) peut être fermée par une deuxième vanne (10) commandée par l'installation de mesure (6).

10. Appareil (14) selon la revendication 8 ou 9, **caractérisé en ce que** la chambre de mesure (5) communique en vue de la circulation avec l'environnement (3) du boîtier (1) par une conduite de sortie de gaz (11), pouvant en particulier être fermée par une troisième vanne (12).

11. Appareil (14) selon l'une des revendications 8 à 10, **caractérisé en ce qu'**au moins une pompe (13) est prévue pour pomper au moins indirectement le gaz de la chambre de mesure (5) dans le boîtier (1).

12. Appareil (14) selon la revendication 11, **caractérisé en ce qu'**un côté d'aspiration de la pompe (13) est tourné vers la chambre de mesure (5) du point de vue de la circulation.

13. Appareil (14) selon l'une des revendications 8 à 12, **caractérisé en ce que** la chambre de mesure (5) est disposée à l'intérieur du boîtier (1).

14. Appareil (14) selon l'une des revendications 8 à 13, **caractérisé en ce que** l'installation de mesure (6) présente au moins un capteur de pression (16) qui mesure une différence de pression entre le gaz dans le boîtier (1) et l'environnement (3).

15. Appareil (14) selon l'une des revendications 8 à 14, **caractérisé en ce que** l'appareil (14) est un véhicule sans pilote.
